# FASCICULE DE BREVET EUROPEEN

(11) **EP 0 478 470 B1**
(45) Date de publication et mention de la délivrance du brevet: **05.07.1995**
(21) Numéro de dépôt: 91420323.7
(22) Date de dépôt: 13.09.1991
(51) Int. Cl.: A61B 17/60

(54) **Ligament synthétique vertébral**
Synthetisches Band für die Wirbelsäule
Vertebral synthetic ligament

(30) Priorité: 21.09.1990 FR 9011900
(43) Date de publication de la demande: 01.04.1992
(73) Titulaire: Commarmond, Jacques, F-71100 Saint Remy (FR); IMPACT, F-01800 Charnoz (FR)
(72) Inventeur: Commarmond, Jacques, F-71100 Saint Remy (FR)
(74) Mandataire: Laurent, Michel

(56) Documents cités:
- EP-A- 322 334
- EP-A- 381 588
- DE-A- 2 821 678
- US-A- 4 743 260

## Description

L'invention concerne un ligament synthétique vertébral, fixé par des vis pédiculaires solidarisant deux vertèbres contigues.

L'invention est particulièrement adaptée à un ligament synthétique vertébral, destiné à être disposé entre chaque vertèbre lombaire, ainsi qu'entre la dernière lombaire et le sacrum, notamment les quatrième et cinquième vertèbres lombaires (L4 et L5) ou entre la cinquième lombaire et la première sacrée (L5 et S1).

L'invention concerne plus particulièrement un ligament vertébral synthétique modulaire, destiné à être placé consécutivement sur les vertèbres lombaires en se chevauchant à la manière de marches d'escalier.

Ce ligament synthétique vertébral, destiné à être fixé par des vis pédiculaires sur deux vertèbres de la colonne vertébrale, se caractérise en ce qu'il se compose :
- d'un premier et d'un second oeillets creux, en forme générale de diabolo, comprenant chacun dans l'ordre :
   . un logement concave destiné à recevoir la tête d'une des vis pédiculaires de fixation ;
   . un corps présentant un étranglement ;
   . un bossage convexe, complémentaire du logement concave, destiné à être dirigé vers la vertèbre ;
- d'un ligament synthétique proprement dit, en un matériau textile souple, constitué :
   . d'un enroulement primaire longitudinal disposé en huit entre les étranglements des deux oeillets ;
   . un enroulement secondaire transversal disposé en spires jointives autour de l'enroulement primaire.

La manière dont l'invention et les avantages qui en découlent ressortiront mieux de l'exemple de réalisation qui suit à l'appui des figures annexées.

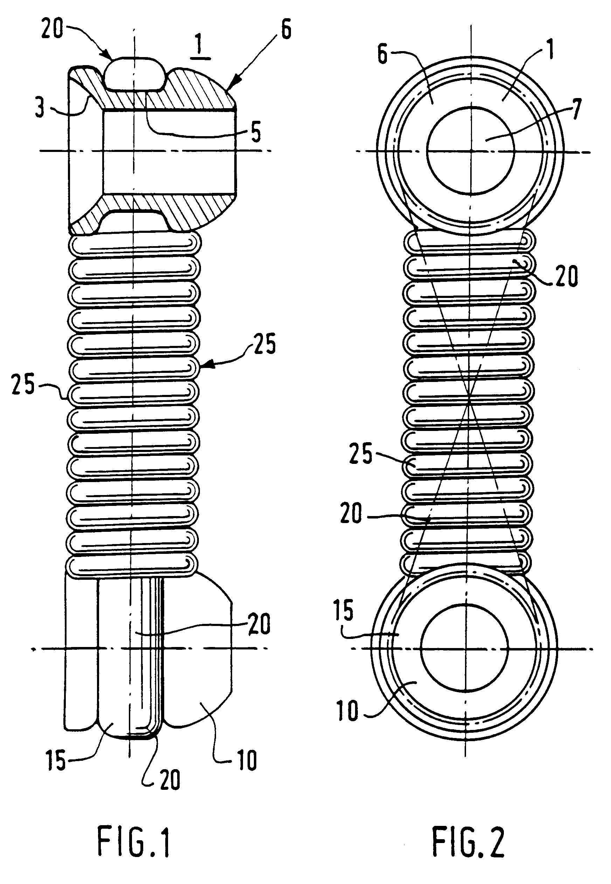

Les figures 1 et 2 montrent une vue en coupe d'un ligament synthétique vertébral conforme à l'invention, respectivement vu en coupe et vu de face.

Les figures 3 et 4 montrent un oeillet caractéristique de l'invention respectivement vu en coupe et vu de face.

La figure 5 montre une série de trois ligaments synthétiques vertébraux mis en place conformément à l'invention.

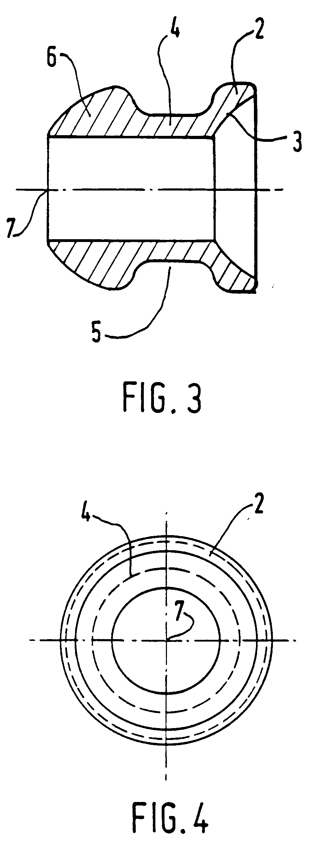

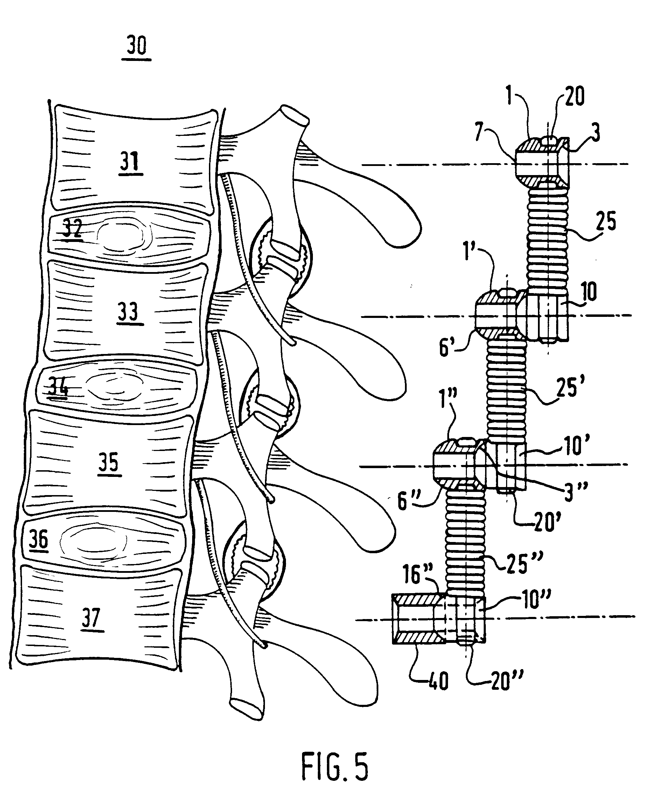

Selon l'invention (voir figures 3 et 4), le ligament comprend un premier (1) et un second (10) oeillets creux en métal (acier inoxydable, alliage de titane, acier chrome/cobalt) en forme générale de diabolo. Chacun de ces deux oeillets (1,10) comprend sur la face externe (2) un logement concave (3), destiné à recevoir la tête de la vis de fixation non représentée. Cette vis, par exemple de type Muller ou Maconnor, est destinée à fixer l'oeillet sur les apophyses articulaires (voir figure 5). Ces vis à corticales, traversent les pédicules pour se loger dans le corps vertébral. Dans une variante, ce logement concave (3) peut recevoir un autre oeillet identique consécutif, de manière à jouer le rôle de bague anti-cisaillement.

Cette face (2) est associée à un corps (4) dans lequel est ménagé un étranglement médian (5), associé à son tour à un bossage convexe (6), complémentaire du logement concave (3). Ce bossage convexe (6) est comme déjà dit, soit destiné à venir s'encastrer dans l'apophyse articulaire en y ayant pratiqué préalablement un petit lamage pour jouer le rôle de bague anti-cisaillement, ou dans le logement concave (3) d'un oeillet consécutif (voir figure 5). Un canal central (7) permet le passage de la vis de fixation. Pour éviter des blessures et faciliter l'articulation de l'ensemble, les différents angles de l'oeillet sont arrondis.

Selon une autre caractéristique de l'invention, le ligament comprend un ligament synthétique proprement dit en un matériau textile souple, constitué d'un enroulement primaire (20), longitudinal, disposé en huit entre les étranglements (5) et (15) de deux oeillets (1,10) successifs. Cet enroulement primaire (20) longitudinal est recouvert à son tour par un enroulement secondaire transversal (25) disposé en spires jointives autour de cet enroulement primaire longitudinal (20). L'enroulement primaire longitudinal (20) confère à l'ensemble la rigidité en traction. En revanche, l'enroulement secondaire transversal (25) se comporte comme une cale et donne au ligament la raideur en compression.

En pratique, ces deux enroulements primaire longitudinal (20) et secondaire transversal (25), sont constitués par une tresse en fils multifilamentaires de polyester rétractable. De la sorte, par un simple étuvage au four sec, on provoque la rétraction des filaments (de 12 % par exemple), ce qui donne à l'ensemble sa raideur et la dimension souhaitée. De préférence, la section de la tresse constituant l'enroulement primaire (20) est inférieure à la tresse externe secondaire (25), de manière à éviter le chevauchement des spires jointives de l'enroulement secondaire (25).

La figure 5 montre la manière dont le ligament caractéristique de l'invention peut être mis en place pour le renforcement d'une colonne vertébrale, désignée par la référence générale (30), dans laquelle les références (31,33,35,37) désignent des vertèbres, par exemple (L4,L5 et S1) et les références (32,34,36) les disques. Le premier ensemble (1,25,10) est fixé à l'aide vis pédiculaires non représentées, sur les apophyses articulaires en traversant les pédicules pour se loger dans le corps vertébral (31,33). Le second ensemble (1',25',10') renforce les vertèbres suivantes (33,35), l'oeillet de base (10) du premier ensemble coopérant avec le premier oeillet (1′) de l'ensemble suivant comme montré à la figure. De même, le troisième ensemble (20'',25'',10'') coopère avec le second ensemble pour renforcer les vertèbres (35,37). On comprend que les parties hémisphériques mâle (6) et femelle (3) des oeillets s'imbriquent les unes dans les autres, pour constituer un ensemble compact permettant un ajustement avec un léger rotulage. De même, l'étranglement (4), qui reçoit l'enroulement primaire (20) longitudinal, permet grâce à cette forme de diabolo, d'immobiliser cet enroulement primaire (20) en translation sans le fatiguer, ce qui évite des ruptures de fatigue (à la longue en cours de porter).

Dans une variante montrée à la figure 5, l'oeillet caractéristique peut coopérer avec une entretoise (40) de réhaussement, de même dimension et de même hauteur que l'oeillet caractéristique (10'').

La raideur du ligament conforme à l'invention est appréciable aussi bien en traction qu'en compression.

Dans une forme de réalisation pratique, on a obtenu de bons résultats avec des oeillets dont le diamètre et la longueur sont voisines de dix millimètres, percés d'un canal (7) d'environ sept millimètres et dont les rayons de courbure du logement concave (3) et du bossage convexe (6) sont égaux à cinq millimètres, ce qui permet de recevoir des vis de Muller de 4,5 mm de diamètre ou Maconna de 5 mm. L'enroulement primaire longitudinal (20) est constitué par soixante passages en huit d'un fil de polyester haute ténacité rétractable de 1000 deniers. Cet enroulement primaire (20) est ensuite recouvert d'une tresse à spires jointives de trois millimètres de diamètre, réalisée à partir du même fil de polyester haute ténacité rétractable 1000 deniers sur six fuseaux, de manière à former un enroulement secondaire (25). La tresse externe (25) formant enroulement secondaire est fermement enroulée autour de l'enroulement primaire interne (20), de manière à fermer par ses extrémités les boucles de l'enroulement primaire. Une fois cet ensemble réalisé, on le traite de manière connue à la chaleur pour provoquer la rétraction des fils de polyester. Cela donne à l'ensemble l'élasticité requise en traction avec une faible amplitude jusqu'à l'ouverture des bouts du vide de l'enroulement primaire qui est en effet pratiquement inextensible.

L'espacement entre deux oeillets (1,10) du même ensemble est modulaire, par exemple vingt-cinq, trente, trente-cinq millimètres.

Dans ces conditions, on a observé que l'amplitude maximale entre l'allongement de ce ligament en cyphose et sa compression en lordose, est d'environ deux millimètres.

Le ligament synthétique vertébral conforme à l'invention présente de nombreux avantages par rapport aux solutions connues à ce jour. On peut citer :
- l'implantation pédiculaire solide et possible après laminectomie complète ;
- la souplesse préservant la fixation pédiculaire associée à une raideur en traction et en compression, limitant la cyphose-lordose entre deux vertèbres et par là, le cisaillement discal ;
- la possibilité d'implantations modulaires permettant d'étendre à volonté le nombre d'étages inter-vertébraux à fixer, les différentes tailles proposées pour chaque module s'adaptant à chaque situation anatomique.

## Revendications

1. Ligament synthétique vertébral (1), destiné à être fixé par des vis pédiculaires sur deux vertèbres (31,33) de la colonne vertébrale (30), caractérisé en ce qu'il se compose :
- d'un premier (1,1',1'') et d'un second (10,10',10'') oeillets creux, en forme générale de diabolo, comprenant chacun dans l'ordre :
. un logement concave (3), destiné à recevoir la tête d'une des vis pédiculaires de fixation ;
. un corps (4) présentant un étranglement (5) ;
. un bossage convexe (6) complémentaire du logement concave (3), destiné à être dirigé vers la vertèbre (31-37) ;
- d'un ligament synthétique proprement dit, en un matériau textile souple, constitué :
. d'un enroulement primaire (20) longitudinal, disposé en huit entre les étranglements (5,15) des deux oeillets (1,10) ;
. d'un enroulement secondaire transversal (25), disposé en spires jointives autour de l'enroulement primaire (20).

2. Ligament synthétique vertébral selon la revendication 1, caractérisé en ce que les oeillets (1,10) sont en métal.

3. Ligament synthétique vertébral selon la revendication 1, caractérisé en ce que les oeillets (1,10) monobloc sont en acier inoxydable, en alliage de titane, en acier chrome/cobalt.

4. Ligament synthétique vertébral selon la revendication 1, caractérisé en ce que l'enroulement primaire longitudinal interne (20) et l'enroulement secondaire transversal externe (25), sont réalisés en une tresse en multifilaments en polyester rétracté.

5. Ligament synthétique vertébral selon l'une des revendications 1 à 4, caractérisé en ce que l'ensemble comporte également une entretoise (40), qui prend appui d'un côté sur la vertèbre (37) et de l'autre sur le bossage convexe (16'') dans l'oeillet (10'').

## Claims

1. Artificial ligament (1) for the spine, intended to be fixed by pedicle screws on two vertebrae (31, 33) of the spinal column (30), characterised in that it consists of :
- a first hollow eyelet (1, 1', 1'') and a second hollow eyelet (10, 10', 10''), of general diabolo shape, each one comprising in order :
·
· a concave seat (3) intended to receive the head of one of the pedicle fixation screw ;
· a body (4) having a narrowed area (5) ;
· a convex hump (6) complementing the concave seat (3) and intended to be directed towards the vertebra (31-37) ;
- an artificial ligament proper, made of a flexible textile material, consisting of :
· a longitudinal primary winding (20) arranged in figure of eight formation between the narrowed areas (5, 15) of the two eyelets (1, 10) ;
· a transverse secondary winding (25) arranged in contiguous spirals around the primary winding (20).

2. Artificial ligament for the spine according to claim 1, characterised in that the eyelets (1, 10) are made of metal.

3. Artificial ligament for the spine according to claim 1, characterised in that the monobloc eyelets (1, 10) are made of stainless steel, titanium alloy, chromium steel/cobalt.

4. Artificial ligament for the spine according to claim 1, characterised in that the inner longitudinal primary winding (20) and the outer transverse secondary winding (25) are formed by a braiding of multifilaments of retracted polyester.

5. Artificial ligament for the spine according to claim 1 to 4, characterised in that the assembly also comprises a strut (40) which bears on one side on the vertebra (37) and on the other side on the convex hump (16'') in the eyelet (10'').

## Patentansprüche

1. Künstliches Wirbelband, das dazu vorgesehen ist, mittels Pedikelschrauben an zwei Wirbeln (31, 33) der Wirbelsäule (30) befestigt zu werden, dadurch gekennzeichnet, daß es wie folgt aufgebaut ist, nämlich
- aus einer ersten (1, 1', 1'') und einer zweiten (10, 10', 10'') hohlen Hülse in der allgemeinen Form eines Diabolo, von der jede folgendes aufeinanderfolgend enthält:
. eine konkave Aufnahme (3), die dazu vorgesehen ist, den Kopf einer der Pedikelbefestigungsschrauben aufzunehmen;
. einen eine Einschnürung (5) aufweisenden Körper (4);
. einen zur konkaven Aufnahme (3) komplementären konvexen Anguß (6), der auf den Wirbel (31 - 37) ausgerichtet ist;
- sowie aus dem genannten künstlichen Band aus einem flexiblen textilen Material, gebildet aus:
. einer ersten, längsverlaufenden Wicklung (20), die in Form einer Acht um die Einschnürungen (5, 15) der beiden Hülsen (1, 10) angeordnet ist;
. sowie einer zweiten, guerverlaufenden Wicklung (25), die in aneinanderliegenden Windungen um die erste Wicklung (20) herum angeordnet ist.

2. Künstliches Wirbelband nach Anspruch 1, dadurch gekennzeichnet, daß die Hülsen (1, 10) aus Metall bestehen.

3. Künstliches Wirbelband nach Anspruch 1, dadurch gekennzeichnet, daß die einstückigen Hülsen (1, 10) aus Edelstahl, einer Titanlegierung oder einem Chrom-Kobalt-Stahl bestehen.

4. Künstliches Wirbelband nach Anspruch 1, dadurch gekennzeichnet, daß die innere, längsverlaufende erste Wicklung (20) und die äußere, querverlaufende zweite Wicklung (25) aus einem Multifilamentflechtwerk aus geschrumpften Polyesterfasern bestehen.

5. Künstliches Wirbelband nach einem der Ansprüche 1 bis 4, dadurch gekennzeichnet, daß der Zusammenbau außerdem eine Distanzhülse (40) aufweist, die sich einerseits auf dem Wirbel (37) und andererseits auf dem konvexen Anguß (16'') an der Hülse (10'') abstützt.
